# EUROPEAN PATENT APPLICATION

(11) **EP 4 112 715 A1**
(43) Date of publication of application: **04.01.2023**
(21) Application number: 21305911.6
(22) Date of filing: 01.07.2021
(51) Int. Cl.: C12N 1/20, A23C 9/00, C12R 1/00

(54) **BIFIDOBACTERIA FOR USE IN PREPARATION OF FERMENTED PRODUCTS**

(71) Applicant: Compagnie Gervais Danone, 75009 Paris (FR)
(72) Inventor: BONIFACE-GUIRAUD, Audrey, 91340 LES ULIS (FR); GARAULT, Peggy, 91310 MONTLHERY (FR); FAURIE, Jean-Michel, 78350 JOUY-EN-JOSAS (FR); GALAND, Valentin, 13012 MARSEILLE (FR)
(74) Representative: Gevers & Orès

(57) **Abstract**

The present invention relates to novel strains of *Bifidobacteria,* compositions comprising said bacteria and to methods for the preparation of such compositions.

## Description

### Field of the invention

The present invention relates to novel *Bifidobacteria,* compositions comprising said bacteria and methods for the preparation of such compositions.

### Technical background

There is increased interest in plant-based diets among mainstream consumers who consider themselves vegan, vegetarian or flexitarian. To cater to the dietary needs of such consumers a wide variety of plant-based analogues or alternatives to non-vegan food products are increasingly available. These include plant-based dairy alternatives such as milks, yogurts, cheeses & frozen desserts. The formulation of such products to provide a sensory and/or nutritional equivalent remains challenging. This is especially the case in the formulation of "probiotic" food products which are also increasingly popular with consumers. According to a definition approved by a joint Food and Agriculture Organization of the United Nations/World Health Organization (FAO/WHO) expert Consultation on Health and Nutritional properties of powder milk with live lactic acid bacteria in 2001, probiotics are "live microorganisms which when administered in adequate amounts confer a health benefit on the host". Probiotic bacteria have been described among species belonging to the genera *Lactobacillus, Bifidobacterium, Streptococcus* and *Lactococcus,* commonly used in the dairy industry. However, the addition of probiotic species, especially in the context of fermented food products can be challenging as they can introduce undesirable flavours or off-notes to products.

The use of probiotic species in the preparation of plant-based dairy alternatives is known in the art. US6699517, which is incorporated by reference herein, teaches the fermentation of vegetal bases using *S. thermophilus* CNCM I-1520 and various probiotic species (*L. plantarum; L. casei; Bifidobacteria*).

WO2021028725 teaches that the widely used probiotic species *Bifidobacteria,* are associated with vinegary off-notes in plant-based yogurt alternatives, due to the production of acetic acid. A common solution for addressing tart or acidic notes is the addition of sugars or non-nutritive sweeteners. However, increasing consumer awareness of the benefits of a low-sugar diet together with a trend towards what the consumer perceives as "natural" products has led to a need for the development of fermented milk products with a reduced sugar content that use natural sweetening agents (e.g. stevia).

The use of sweetness enhancing strains of yogurt cultures of *S. thermophilus* and *L. Bulgaricus* strains has also been proposed as a means of increasing the sweetness of fermented milk products (WO 2013/160413; WO 2011/026863).

Nevertheless, it remains challenging to reduce the amount of sugar required for the preparation of fermented products while ensuring a product having good organoleptic properties, low amounts of added sugar and a so-called "clean label" (a short ingredient list, using as many consumer recognizable ingredients as possible). Moreover, the acidity of fermented products can also be increased after the lactic acid fermentation by post acidification due to the continued consumption of sugars by the bacteria.

Accordingly, there remains a need in the art to find a healthy means of improving the taste of *Bifidobacteria* fermented products, particularly in plant-based products.

### Summary of the invention

The present invention follows from the unexpected finding that novel strains of *Bifidobacteria* have surprising metabolic properties. The *Bifidobacteria* of the invention do not metabolize sucrose whilst maintaining the ability to metabolize raffinose and stachyose. Additionally, these strains are exceptionally useful in the preparation of a diverse range of fermented food products based on their versatile ability to ferment and acidify both dairy and plant matter. Accordingly, these strains allow the preparation of probiotic-type fermented products with a reduced requirement for added sugar. This is especially advantageous in the context of plant-based yogurt alternatives such as soy based products where the *Bifidobacteria* may out compete lactic acid homofermentative yogurt cultures and result in "vinegary" tasting products.

Accordingly, the present invention provides compositions comprising sucrose negative, stachyose and raffinose positive *Bifidobacteria* and methods for the preparation thereof. Furthermore, the present invention also provides *B. breve* or *B. longum* strains deposited at the CNCM.

### Detailed description of the invention

As used herein the term "sucrose negative" (or Suc-) shall be taken to mean a bacterium that does not have the ability to metabolize sucrose as a source for cell growth or maintaining cell viability. According to the present invention, a Suc- *Bifidobacteria* strain is not able to grow in a medium containing sucrose as the sole source of carbon. Conversely, the term "sucrose positive" (or Suc+) means the ability of a bacterium to metabolize sucrose or to grow in a medium containing sucrose as a sole source of carbon.

These phenotypes can be determined using techniques well known by a skilled person in the art. For example, these phenotypes can be assessed by inoculating a MRS no-sugar media supplemented with sucrose cultured at 37°C for 24 hours. Alternatively the metabolic profile of a bacterial strain can be determined using commercially available kits such as the API gallery (BioMerieux).

The above definition also applies to the phenotypes related to other carbohydrates fermentable by bacteria, in particular lactose, galactose, glucose or raffinose.

Accordingly, the term "raffinose positive" (or Raf+) shall be taken to mean a bacterium that does have the ability to metabolize raffinose as a source for cell growth or maintaining cell viability.

Accordingly, the term "stachyose positive" (or Sta+) shall be taken to mean a bacterium that does have the ability to metabolize stachyose as a source for cell growth or maintaining cell viability.

As used herein, the term "ppm" shall be taken to mean "parts per million" One gram in 1 liter is 1000 ppm and one thousandth of a gram (0.001g) in 1 liter is one ppm.

As used herein the term "x% (w/w)" is equivalent to "x g per 100 g".

In the context of this application, the term "at least" also includes the starting point of the open range. For example, an amount of "at least 95.00 % w/w" means any amount equal to 95.00 percentage by weight or above.

In the context of this application, the term "about" defines a range of plus or minus 10% of the cited value. For example, an amount of "about 20 weight %" means any amount within the range of 18.00 to 22.00 weight %.

As used herein, the term "plant-based" shall be taken to mean a composition or product which does not comprise animal or animal-derived (e.g. mammal milk) matter.

As used herein, the adjective "dairy" shall be taken to mean a composition or product comprises or consists of mammalian milk matter, i.e. the lacteal secretion obtainable by milking. As used herein the terms "dairy composition", "milk-based composition" or "dairy product" shall be taken to mean a product or composition comprising essentially of or consisting of milk or milk components and optionally further ingredients.

As used herein, the terms "-free" or "free from" shall be taken to mean a composition or product which preferably does not contain a given substance but where trace amounts or contaminants thereof may be present.

As used herein, the term "added sugar" shall refer to sugars that are added during the processing of foods (e.g. plant matter processed to provide a vegetal base) as opposed to sugars naturally occurring in said foods. Added sugars include sugars (free, mono- and disaccharides), sugars from syrups and honey, and sugars from concentrated fruit or vegetable juices that are in excess of what would be expected from the same volume of 100 percent fruit or vegetable juice of the same type.

As used herein, the term "fermented plant-based" shall be taken to mean a product or composition that is the product of the acidifying fermentation of a plant-based composition by a starter culture of fermenting microorganisms, in particular bacteria, preferably lactic acid bacteria.

As used herein the term "fermented dairy" shall be taken to mean a product or composition that is the product of the acidifying fermentation of a milk-based composition by a starter culture of fermenting microorganisms, in particular bacteria, preferably lactic acid bacteria. As used herein the term "fermented milk" shall be taken to mean a product or composition derived from milk by the acidifying action of at least one lactic acid bacterium. Accordingly, as used herein a fermented dairy product can thus be a fermented milk, such as a yoghurt (e.g. a set, stirred or drink yogurt), or a fresh cheese such as a white cheese or a *"petit-Suisse".* It can be also be a strained fermented milk such as a strained yoghurt (e.g. a concentrated or Greek-style yoghurt).

As used herein, the terms plant-based alternative, analogue or substitute shall be taken to mean a plant-based food or beverage composition that is formulated to simulate the organoleptic and/or nutritional qualities of a non plant-based product. Accordingly, a "plant-based fermented milk alternative" shall be taken to mean a plant-based food or beverage composition that is formulated to simulate the organoleptic and/or nutritional qualities of fermented dairy milk. A "plant-based yogurt" shall be taken to mean a plant-based food or beverage composition that is formulated to simulate the organoleptic and/or nutritional qualities of fermented dairy yogurt.

The terms "fermented milk" and "yogurt" or "yoghurt" are given their usual meanings in the field of the dairy industry, that is, products suitable for human consumption and originating from acidifying lactic fermentation of a milk substrate. These products can contain secondary ingredients such as fruits, vegetables, sugar, etc. The expression "fermented milk" may be used to refer to fermented milks other than yogurts e.g. "Kefir", "Kumtss", "Lassi", "Dahi", "Leben", "Filmjolk", "Villi", "Acidophilus milk".

The term "yogurt" or "yoghurt" as used herein shall be taken to mean fermented milk obtained by the acidifying lactic fermentation of specific thermophilic lactic acid bacteria such as *Lactobacillus delbrueckii* subsp. *bulgaricus* and *Streptococcus thermophilus* (also referred to as *Streptococcus salivarius* subsp. *thermophilus*), which must be in the living state in the finished product at a minimum CFU. In certain countries, regulations allow the addition of further lactic acid bacteria to yoghurt such as but not limited to strains of *Bifidobacterium* and/or *Lactobacillus acidophilus* and/or *Lactobacillus casei.* These additional lactic acid bacteria strains are intended to impart various properties to the finished product, such as that of providing organoleptic qualities, favoring equilibrium of intestinal flora or modulating the immune system.

As used herein the term "strained fermented food product" shall be taken to mean a food product which has been subjected to a post-fermentation separation process.

As used herein the term "spoonable" shall be taken to mean a solid or semi-solid that may be consumed by means of a spoon or other utensil.

As used herein the term "fermentation" shall be taken to mean the metabolism of a substance by microorganisms, e.g. bacteria, yeasts, or other microorganisms.

As used herein the term "cfu" or "CFU" shall be taken to be an abbreviation of the term "colony forming unit".

As used herein the term "CNCM I-" followed by a 4 digit number shall be taken to refer to a strain deposited at the Collection Nationale de Cultures de Microorganismes (CNCM) 25 rue du Docteur Roux, 75724 Paris Cedex 15, France under the Budapest Treaty with an accession number corresponding to said 4 digit number.
As used herein reference to a bacterial strain or species shall be taken to include functionally equivalent bacteria derived therefrom such as but not limited to mutants, variants or genetically transformed bacteria. These mutants or genetically transformed strains can be strains wherein one or more endogenous gene(s) of the parent strain has (have) been mutated, for instance to modify some of their metabolic properties (*e.g.,* their ability to ferment sugars, their resistance to acidity, their survival to transport in the gastrointestinal tract, their post-acidification properties or their metabolite production). They can also be strains resulting from the genetic transformation of the parent strain to add one or more gene(s) of interest, for instance in order to give to said genetically transformed strains additional physiological features, or to allow them to express proteins of therapeutic or prophylactic interest that one wishes to administer through said strains. These mutants or genetically transformed strains can be obtained from the parent strain by means of conventional techniques for random or site-directed mutagenesis and genetic transformation of bacteria, or by means of the technique known as "genome shuffling". In the present text, strains, mutants and variants derived from a parent species or strain will be considered as being encompassed by reference to said parent species or strain, *e.g.* the phrases *"B. breve"* and "CNCM I-5542" " or *"B. breve"* and "CNCM I-5543" or *"B. longum"* and "CNCM I-5544" shall be taken to include strains, mutants and variants derived therefrom. Accordingly, as used herein reference to a bacterial strain specified by an accession or deposit number shall be taken to encompass variants thereof having at least 95 % identity (see: Stackebrandt & Goebel, 1994, Int. J. Syst. Bacteriol. 44:846-849). In a particularly preferred embodiment, said variant has at least 97 % identity with the 16S rRNA sequence of said specified strain, more preferably at least 98 % identity, more preferably at least 99 % or more identity.

As used herein the term "substantially pure" when used in reference to a bacterial strain refers to the percent of said bacterial strain relative to the total micro-organism content. Substantially pure can be at least about 99.99%, at least about 99.90%, at least about 99.50%, at least about 99.00%, at least about 95.00%, at least about 90.00%, at least about 85.00%, or at least about 75.00%.

As used herein, a "lactic acid bacterium" is a Gram-positive, acid-tolerant, generally non-sporulating and non-respiring, either rod- or cocci-shaped bacterium that is able to ferment sugars into lactic acid.

The present invention relates to novel strains of *Bifidobacteria* compositions comprising said strains and to methods for the preparation of such compositions.

### Bifidobacteria

In a first aspect the present invention provides sucrose negative, stachyose and raffinose positive *Bifidobacteria.* In a first embodiment the present invention provides the strain *B. breve* CNCM 1-5542. This strain has been deposited at the Collection Nationale de Cultures de Microorganismes (CNCM) (Institut Pasteur, 25 Rue du Docteur Roux, 75724 Paris Cedex 15, France) under the Budapest Treaty on July 16^{th} 2020 under reference number CNCM 1-5542. The deposit was made in accordance with the Budapest Treaty on the International Recognition of the Deposit of Microorganisms for the Purposes of Patent Procedure, as provided therein the applicant requests that a sample of the deposited micro-organisms only be made available to an independent expert, until the date on which the patent may be granted. In one embodiment the present invention provides the isolated strain *B. breve* CNCM 1-5542, preferably said isolate is substantially pure.

In a second embodiment the present invention provides the strain *B. breve* CNCM 1-5543. This strain has been deposited at the Collection Nationale de Cultures de Microorganismes (CNCM) (Institut Pasteur, 25 Rue du Docteur Roux, 75724 Paris Cedex 15, France) under the Budapest Treaty on July 16^{th} 2020 under reference number *CNCM I-5543.* The deposit was made in accordance with the Budapest Treaty on the International Recognition of the Deposit of Microorganisms for the Purposes of Patent Procedure, as provided therein the applicant requests that a sample of the deposited micro-organisms only be made available to an independent expert, until the date on which the patent may be granted. In one embodiment the present invention provides the isolated strain *B. breve CNCM I-*5543, preferably said isolate is substantially pure.

In a third embodiment the present invention provides the strain *B. longum* CNCM 1-5544. This strain has been deposited at the Collection Nationale de Cultures de Microorganismes (CNCM) (Institut Pasteur, 25 Rue du Docteur Roux, 75724 Paris Cedex 15, France) under the Budapest Treaty on July 16^{th} 2020 under reference number CNCM 1-5544. The deposit was made in accordance with the Budapest Treaty on the International Recognition of the Deposit of Microorganisms for the Purposes of Patent Procedure, as provided therein the applicant requests that a sample of the deposited micro-organisms only be made available to an independent expert, until the date on which the patent may be granted. In one embodiment the present invention provides the isolated strain *B. longum* CNCM I-5544, preferably said isolate is substantially pure.

### Compositions of the invention

In a second aspect the present invention provides compositions comprising sucrose negative, stachyose and raffinose positive *Bifidobacteria* ("*Bifidobacteria* of the invention"). In embodiments the sucrose negative, stachyose and raffinose positive *Bifidobacteria* are selected from the group consisting of *Bifidobacterium animalis* (for example *Bifidobacterium animalis* subsp. *animalis* or *Bifidobacterium animalis* subsp. *lactis*), *Bifidobacterium longum, Bifidobacterium breve* and *Bifidobacterium bifidum.*

Preferably the sucrose negative, stachyose and raffinose positive *Bifidobacteria* are *B. breve* or *B. longum,* more preferably selected from group consisting of CNCM 1-5542, CNCM 1-5543, CNCM I-5544 and/or combinations thereof.

Preferably, the composition comprises at least 10⁵, preferably at least 10⁶, more preferably at least 10⁷ and most preferably at least 10⁸ colony forming unit (CFU) sucrose negative, stachyose and raffinose positive *Bifidobacteria* per gram (g) of composition according to embodiments of the invention.

In embodiments, the composition comprises 10⁵ to 10¹² colony forming unit (CFU) sucrose negative, stachyose and raffinose positive *Bifidobacteria* per gram (g) of composition according to embodiments of the invention. In further embodiments, the composition comprises 10⁶ to 10¹¹ colony forming unit (CFU) *Bifidobacteria* selected from the group consisting of CNCM 1-5542, CNCM 1-5543, CNCM I-5544 and/or combinations thereof per gram (g) of composition according to embodiments of the invention.

Advantageously, the composition is a manufactured composition.

In an embodiment, in addition to the *Bifidobacteria* selected from the group consisting of CNCM 1-5542, CNCM 1-5543, CNCM I-5544 and/or combinations, the composition may comprise a medium. The medium may be a milk base fermented or not fermented, a vegetable base fermented or not fermented or any other medium for example a culture medium.

The bacterium as provided herein is suitable for use in edible compositions, accordingly in one embodiment the present invention provides a composition suitable for human consumption or ingestion, preferably by oral means. Accordingly the composition comprises or consists of comestible matter. It is particularly preferred that the compositions of embodiments of the invention are substantially free of pathogenic or toxicogenic matter. The composition according to embodiments of the invention may be a medicament or pharmaceutical composition. In a particularly preferred embodiment the composition according to the invention may be a non-therapeutic composition, preferably a nutraceutical composition, a nutritional composition and/or a food composition. It is particularly preferred that the food composition is a food product, preferably fermented food product, preferably a fermented plant-based or dairy food product. Further compositions according to embodiments of the invention also include food additives, food ingredients, nutritional formulas, baby foods, infant milk formulas and infant follow-on formulas.

The composition may comprise further additional strains of lactic acid bacteria; typically 1, 2, 3, 4 or more additional strains. Examples of lactic acid bacteria that can be used include but are not limited to *Lactobacilli* (for example *Lactobacillus acidophilus, Lactobacillus buchneri, Lactobacillus delbrueckii,* in particular *L. delbrueckii* subsp. *bulgaricus* or *lactis* or *delbrueckii, Lactobacillus casei, Lactobacillus plantarum, Lactobacillus reuteri, Lactobacillus johnsonii, Lactobacillus helveticus, Lactobacillus brevis, Lactobacillus rhamnosus*); *Lactococci* (for example *Lactococcus lactis,* typically *Lactococcus lactis* subsp. *lactis* or *Lactococcus lactis* subsp. *cremoris*). Preferably the composition further comprises *Lactobacillus* and/or *Streptococcus.* For the preparation of yogurt or plant-based alternatives thereto, the composition typically comprises *Lactobacillus bulgaricus* (also referred to as *Lactobacillus delbrueckii* subsp. *bulgaricus*) and *Streptococcus thermophilus,* optionally with additional microorganisms such as but not limited to probiotic species or other species that may provide desirable organoleptic or other qualities to the composition, *e.g.* further strains of *Lactococcus lactis.*

In embodiments the compositions of the invention comprise raffinose. In an embodiment raffinose is present in an amount between 0.1 and 50 mg/100g by weight of composition, for example, or from 2 and 40 mg/100g, or from 3 and 30 mg/100g.

In embodiments the compositions of the invention comprise stachyose. In an embodiment stachyose is present in an amount between 0.1 and 350 mg/100g by weight of composition, for example, or from 2 and 300 mg/100g, or from 3 and 250 mg/100g.

In embodiments the compositions of the invention comprise sucrose. In an embodiment sucrose is present in an amount between 1% and 15% by weight of composition, for example, or from 1% to 10%, or from 2% to 10% or from 3% to 10% or from 5% to 10% or from 5% to 9% or from 5% to 8% or from 5% to 7%. In embodiments the sucrose is present fully or partially in the form of added sugar. In embodiments the added sugar is selected from the group consisting of beet sugar, brown sugar, brown rice sugar, cane juice, cane syrup, cane sugar, caramel, coconut sugar, corn syrup solids, corn syrup, refined sugar, confectioner's sugar, date sugar, maple syrup, malt, molasses, raw sugar, sugar, honey, agave and/or combinations thereof.

In embodiments the compositions of the invention comprise stachyose, raffinose and sucrose as described herein.

### Fermented food products

The sucrose negative, stachyose and raffinose positive *Bifidobacteria* of the present invention are particularly suited to the preparation of fermented food products in embodiments a fermented plant-based or dairy food product.

In one embodiment the present invention provides a plant-based food product, preferably a fermented plant-based food product such as a dairy-alternative. The plant-based food products of the invention comprises a vegetal base, preferably fermented vegetal base.

In one embodiment, the vegetal base is an aqueous suspension comprising water and plant-matter selected from the group consisting of legumes, nuts, seeds, cereals and/or combination thereof. Particularly preferred is a base that comprises added sugar, where the total carbohydrate content of the vegetal base is derived from plant-matter selected from the group consisting of legumes, nuts, seeds, cereals and/or combination thereof and added sugar. In embodiments the added sugar is selected from the group consisting of beet sugar, brown sugar, brown rice sugar, cane juice, cane syrup, cane sugar, caramel, coconut sugar, corn syrup solids, corn syrup, refined sugar, confectioner's sugar, date sugar, maple syrup, malt, molasses, raw sugar, sugar, honey, agave and/or combinations thereof.

In other embodiments, the plant-matter comprises legumes, and most preferably, pulse or pulses. In other embodiments, the pulses are selected from the group consisting of split peas, field peas, dry peas, lentil, chickpeas, garbanzo bean, faba beans, konda, navy bean, white navy bean, white pea bean, pea bean, cow pea, horse bean, haricot, pinot bean, mottled bean, small red bean, red Mexican bean, kidney bean, black bean, black turtle bean, cranberry bean, roman bean, speckled sugar bean, lima bean, haba bean, Madagascar bean, green gram, mung bean, green bean, black gram, urad dal, soy and/or lupin. In preferred embodiments, the pulses are pea and/or chickpea.

In other embodiments, the nuts are selected from the group consisting of almonds, cashews, pecans, macadamias, hazelnuts, pistachio, walnuts or combinations thereof.

In other embodiments, the seeds are selected from the group consisting of hemp, pumpkin, quinoa, sesame, tiger nut, flax, chia, sunflower, coconut or combinations thereof.

In other embodiments, said cereals are selected from the group consisting of wheat, rye, spelt, barley, oat, millet, sorghum, rice, teff and combinations thereof.

Processes for the preparation of such suspensions are known in the art and typically comprise mechanical and/or enzymatic disruption of the plant-matter and hydration and/or combination with a solution, followed by mechanical separation of an aqueous fraction from starchy and/or fibrous matter, e.g., by decantering, centrifugation or filtration.

For example, the plant-matter may be milled, ground, soaked, dehulled, mixed with water, optionally enzymatic hydrolysed and/or homogenized *etc.* in order to produce a suitable aqueous composition.

In other embodiments, the plant matter may be a seed or nut butter such as sunflower, sesame, soy, almond, cashew, hazelnut or peanut butter. Processes for the preparation of nut butters typically comprise wet or dry grinding roasted or unroasted nuts to a paste having a particle size suitable for the preparation of nut beverages.

In other embodiments, the plant matter may be a hydrolyzed cereal suspension such as an oat milk or syrup. Processes for the preparation of such cereal suspensions typically comprise mixing an oat material (such as rolled oats, milled oats, oat flour or oatmeal) with water and treated enzymatically by amylases to hydrolyze starch followed by removal of suspended matter.

In one embodiment the present invention provides a dairy composition, preferably a fermented dairy food product. The dairy composition of the invention comprises milk, preferably fermented milk. Preferably the composition comprises at least about 30 % (w/w) milk, more preferably at least about 50% (w/w) milk and even more preferably at least about 70% (w/w) milk. In embodiments, the composition comprises 30 % to 100% (w/w) milk. In embodiments, the composition comprises 50% to 100% (w/w) milk. In embodiments, the composition comprises 70% to 100% (w/w) milk. Preferably said milk is animal milk, goat, ewe, camel, mare or cow milk, and most preferably to cow milk. Preferably said milk(s) are heat-treated, typically pasteurized, to ensure sterility. Preferably said heat treatment is carried out prior to the preparation of the fermented dairy food product.

Preferably said milk comprises one or more of skimmed, partially-skimmed or non-skimmed milk. Preferably said milk or milks may be in liquid, powdered and/or concentrated form. In one embodiment said milk further comprises milk components preferably selected from the group consisting of cream, casein, caseinate (for example calcium or sodium caseinate), whey proteins notably in the form of a concentrate (WPC), milk proteins notably in the form of a concentrate (MPC), milk protein hydrolysates, and mixtures thereof. In one embodiment said mixture further comprises plant and/or fruit juices. In one embodiment said milk or milks may be enriched or fortified with further milk components or other nutrients such as but not limited to vitamins, minerals, trace elements or other micronutrients.

Preferably the fermented food product comprises above about 0.3 g per 100 g by weight free lactic acid, more preferably above about 0.7 g or 0.6 g per 100 g by weight free lactic acid. In embodiments, the composition comprises 0.3 g to 0.7 grams per 100 g by weight free lactic acid.

Preferably the fermented food product comprises a protein content, preferably at least about 2.5%, more preferably at least about 3% or 3.5% (w/w). Preferably the composition has a pH equal to or lower than 5, preferably between about 3 and about 4.5 and more preferably between about 3.5 and about 4.5.

Preferably the fermented food product has a viscosity lower than 200 mPa.s, more preferably lower than 100 mPa.s and most preferably lower that 60 mPa.s, at 10°C, at a shear rate of 64 s⁻¹. In embodiments, the composition has a viscosity range of 1 to 200 mPa.s, 1 to 100 mPa.s, or 1 to 60 mPa.s, at 10°C, at a shear rate of 64 s⁻¹. In embodiments, the composition has a viscosity range of 10 to 200 mPa.s, 10 to 100 mPa.s, or 10 to 60 mPa.s, at 10°C, at a shear rate of 64 s⁻¹. In embodiments, the composition has a viscosity range of 30 to 200 mPa.s, 30 to 100 mPa.s, or 30 to 60 mPa.s, at 10°C, at a shear rate of 64 s⁻¹.

The fermented fermented food product according to embodiments of the invention is preferably a product selected from the group comprising yogurt, set yogurt, stirred yogurt, pourable yogurt, yogurt drink, frozen yogurt, kefir, buttermilk, quark, sour cream, fresh cheese, cheese and plant-based alternatives thereto. In one embodiment the composition according to embodiments of the invention is a drinkable composition, more preferably a fermented milk drink such as but not limited to a yogurt drink, kefir etc plant-based alternatives thereto. In an alternative embodiment the composition according to embodiments of the invention is a composition that is spoonable, such as a set or stirred yogurt or plant-based alternatives thereto.

### Strained fermented food products

In one embodiment the fermented food product is a strained fermented food product. The strained fermented food product preferably has the following contents (% by weight):
- from 8.5% to 11.0% of protein
- from 0.0% to 8.0% of fat, for example from 0.0% to 3.5% or from 3.5% to 8.0%
- optionally from 0.00% to 4.20% of lactose, for example from 2.80% to 4.20%

The pH of the strained fermented food product can for example be of from 3.80 to 4.65.

### Intermediate preparations

In embodiments, the food product further comprises an intermediate preparation. Intermediate preparations are known to the one skilled in the art. They are typically used to modify the taste, mouthfeel and/or texture of a food product, for example of a fermented food product. They can used also to introduce some additives such as nutrients. They typically comprise sweetening agents, flavors, color modifiers, cereals and/or fruit. Intermediate fruit preparations are for example slurries or fruit preparations. Flavors include for example fruit flavors, vanilla flavors, caramel flavors, coffee flavors, chocolate flavors.

Fruit preparations typically comprise fruits, as used herein the term "fruit" refers to any fruit form, including for example full fruits, pieces, purees, concentrates, juices etc.

The intermediate preparation or slurry typically comprises a stabilizing agent, having at least one stabilizer. The stabilizing agent can comprise at least two stabilizers. Such stabilizers are known to the one skilled in the art. They typically help in avoiding phase separation of solids, for examples of fruits or fruits extracts and/or in avoiding syneresis. They typically provide some viscosity to the composition, for example a viscosity (Bostwick viscosity at 20°C) of from 1 to 20 cm/min, preferably of from 4 to 12 cm/min.

The stabilizing system or the stabilizer can for example be a starch, a pectin, a guar, a xanthan, a carrageenan, a locust bean gum, or a mixture thereof. The amount of stabilizing system is typically of from 0.5 to 5% by weight.

The intermediate preparation can typically comprise organoleptic modifiers. Such ingredients are known by the one skilled in the art.

The organoleptic modifiers can be for example sweetening agents different from sugar, coloring agents, cereals and/or cereal extracts.

Examples of sweetening agents are ingredients referred to as High Intensity Sweeteners, such as sucralose, acesulfamK, aspartam, saccharine.

Examples of fruits include for example strawberry, peach, apricot, mango, apple, pear, raspberry, blueberry, blackberry, passion, cherry, and mixtures or associations thereof, such as peach-passion.

The fruits can be for example provided as:
- frozen fruit cubes, for example 10 mm fruit cubes, for example Individual Quick Frozen fruit cubes, for example strawberry, peach, apricot, mango, apple, pear fruit cubes or mixtures thereof,
- Aseptic fruit cubes, for example 10 mm fruit cubes, for example strawberry, peach, apricot, mango, apple or pear fruit cubes or mixtures thereof,
- fruit purees, for example fruit purees concentrated from 2 to 5 times, preferably 3 times, for example aseptic fruit purees, for example strawberry, peach, apricot, mango, raspberry, blueberry or apple fruit purees or mixtures thereof,
- single aseptic fruit purees, for example strawberry, raspberry, peach, apricot, blueberry or apple single aseptic fruit purees or mixture thereof,
- frozen whole fruits, for example Individual Quick Frozen whole fruits, for example blueberry, raspberry or blackberry frozen whole fruits, or mixtures thereof,
- mixtures thereof.

The ingredients and/or components of the intermediate preparation and the amounts thereof can be typically such that the composition has a brix degree of from 1 to 65 brix, for example from 1 to 10 brix, or from 10 to 15 brix, or from 15 to 20 brix, or from 20 to 25 brix, or from 25 to 30 brix, or from 30 to 35 brix, or from 35 to 40 brix, or from 40 to 45 brix, or from 45 to 50 brix, or from 50 to 55 brix, or from 55 to 60 brix, or from 55 to 60 brix, or from 60 to 65 brix.. The Brix degree corresponds to the sugar content of a preparation. Methods for measuring the brix degree are well-known by the person skilled in the art. When measuring the brix degree of a fruit preparation, the fruit preparation is filtered on a sieve of 1mm, and the supernatant (thus, without fruit pieces) is collected.

A fruit preparation can for example comprise fruit in an amount of from 30% to 80% by weight, for example from 50 to 70% by weight.

The intermediate preparation can comprise water. It is mentioned that a part of the water can come from ingredients used to prepare the fruit preparation, for example from fruits or fruit extracts or from a phosphoric acid solution.

The fruit preparation can comprise pH modification agents such as citric acid. The fruit preparation can have a pH of from 2.5 to 5, preferably of from 2.8 to 4.2.

Typically a fruit preparation can be added in an amount of 5-35% by weight with reference to the total amount of composition. In embodiments the composition of the invention comprises up to about 30% (w/w) of said intermediate preparation, *e.g.* up to about 10%, 15%, 20%, 25% (w/w). In one embodiment, the composition according to embodiments of the invention comprise 1% to 30% (w/w) of said intermediate preparation. In alternative embodiments, the composition according to embodiments of the invention comprise 1% to 25% (w/w) of said intermediate preparation. In further alternative embodiments, the composition according to embodiments of the invention comprise 1% to 20% (w/w) of said intermediate preparation. In additional embodiments, the composition according to embodiments of the invention comprise 1% to 15% (w/w) of said intermediate preparation. In further additional embodiments, the composition according to embodiments of the invention comprise 1% to 10% (w/w) of said intermediate preparation.

### Packaged compositions

Preferably the composition or food product, according to embodiments of the invention is provided in a sealed or sealable container containing about 50 g, 60 g, 70 g, 75 g, 80 g, 85 g, 90 g, 95 g, 100 g, 105 g, 110 g, 115 g, 120 g, 125 g, 130 g, 135 g, 140 g, 145 g, 150 g, 200 g, 300 g, 320 g or 500 g or about 1 oz, 2 oz, 3 oz, 4 oz, 5 oz, 6 oz or 12 oz product by weight.

In embodiments, the composition or food product, according to embodiments of the invention is provided in a sealed or sealable container containing about 50 g to 500 g, 60 g to 500 g, 70 g to 500 g, 75 g to 500 g, 80 g to 500 g , 85 g to 500 g, 90 g to 500 g, 95 g to 500 g, 100 g to 500 g, 105 g to 500 g, 110 g to 500 g, 115 g to 500 g, 120 g to 500 g, 125 g to 500 g, 130 g to 500 g, 135 g to 500 g, 140 g to 500 g, 145 g to 500 g, 150 g to 500 g, 200 g to 500 g, 300 g to 500 g, 320 g to 500 g or 500 g product by weight. In embodiments, the composition or food product, according to embodiments of the invention is provided in a sealed or sealable container containing about 1 oz to 12 oz, 2 oz to 12 oz, 3 oz to 12 oz, 4 oz to 12 oz, 5 oz to 12 oz, 6 oz to 12 oz or 12 oz product by weight.

Preferably the composition or food product, according to embodiments of the invention, may be stored, transported and/or distributed at a temperature of from 1°C to 10°C for at least about 30 days, at least about 60 days or at least about 90 days from packaging and remain suitable for consumption.

In embodiments, the compositions or food product of the invention comprise at least 10⁵ cfu/g,more preferably at least 10⁶ cfu/g, such as at least 10⁷ cfu/g, e.g. at least 10⁸ cfu/g, such as at least 10⁹ cfu/g, e.g. at least 10¹⁰ cfu/g, such as at least 10¹¹ cfu/g sucrose negative, stachyose and raffinose positive *Bifidobacteria* per gram of composition. In embodiments, the compositions or food product of the invention comprise 10⁵ to 10¹² or 10⁶ to 10¹⁰ colony forming unit (CFU) sucrose negative, stachyose and raffinose positive *Bifidobacteria* per gram of composition.

Preferably, the composition or food product is a packaged product that comprises at least 10⁶, more preferably at least 10⁷ and most preferably at least 10⁸ colony forming unit (CFU) sucrose negative, stachyose and raffinose positive *Bifidobacteria* per gram (g) of composition or food product according to embodiments of the invention subsequent to storage, transport and/or distribution at a temperature of from 1°C to 10°C for at least about 30 days, at least about 60 days or at least about 90 days from packaging.

In embodiments, the composition or food product is a packaged product that comprises 10⁵ to 10¹² or 10⁶ to 10¹⁰ colony forming unit (CFU sucrose negative, stachyose and raffinose positive *Bifidobacteria* per gram (g) of composition or food product according to embodiments of the invention subsequent to storage, transport and/or distribution at a temperature of from 1°C to 10°C for at least about 30 days, at least about 60 days or at least about 90 days from packaging.

### Inoculum

The bacterium as described herein is useful as starter culture in the preparation of food products, such as fermented food products. Accordingly, in one embodiment the present invention provides an inoculum comprising one or more *Bifidobacteria* selected from the group consisting of CNCM 1-5542, CNCM 1-5543, CNCM I-5544 and/or combinations thereof that is suitable for the preparation of fermented food products. The inoculum of the invention is suitable for the direct inoculation of *B. breve* or *B. longum* into a composition comprising a substrate to provide fermented food products of the invention, typically without the need for a culture step prior to the said direct inoculation.

Typically the inoculum further comprises excipient or carriers, the selection of which is within the scope of the skilled person but may include buffers or culture media. The inoculum may optionally comprise further components such as cryoprotectants, preservatives and/or additives including nutrients such as yeast extracts, cysteine, sugars and vitamins.

Typically the inoculum is for use in the preparation of fermented food product, according in one embodiment the inoculum of the invention may be provided to the food product in quantities of up to about 500 mg/l.

Typically the inoculum is fresh, frozen, dried or lyophilized. The inoculum may be in liquid, dry, spray-dried or solid form. It is particularly preferred that the inoculum is in liquid form. The inoculum may be defrosted and/or dispersed in liquid (e.g. water) prior to inoculation into a substrate.

In embodiments, the inoculum comprises at least 10⁹ cfu, e.g. at least 10¹⁰ cfu, such as at least 10¹¹ cfu *B. breve or B. longum* per gram of inoculum composition. In embodiments, the inoculum comprises 10⁹ to 10¹² colony forming unit (CFU), or more preferably 10¹⁰ to 10¹² colony forming unit (CFU) *B. breve or B. longum* per gram of inoculum.

Preferably the inoculum comprising one or more *Bifidobacteria* selected from the group consisting of CNCM 1-5542, CNCM 1-5543, CNCM I-5544 and/or combinations thereof is substantially pure.

In a further embodiment the present invention provides a mixture or kit of parts of the inoculum of the invention together with inoculum of lactic acid bacteria.

Examples of lactic acid bacteria that can be used include but are not limited to *Lactobacilli* (for example *Lactobacillus acidophilus, Lactobacillus buchneri, Lactobacillus delbrueckii,* in particular *L. delbrueckii* subsp. *bulgaricus* or *lactis or delbrueckii, Lactobacillus casei, Lactobacillus plantarum, Lactobacillus reuteri, Lactobacillus johnsonii, Lactobacillus helveticus, Lactobacillus brevis, Lactobacillus rhamnosus*); *Lactococci* (for example *Lactococcus lactis,* typically *Lactococcus lactis* subsp. *lactis* or *Lactococcus lactis* subsp. *cremoris*). Preferably the inoculum mixture further comprises *Lactobacillus* and/or *Streptococcus.* For the preparation of yogurt, the inoculum mixture typically comprises *Lactobacillus bulgaricus* (also referred to as *Lactobacillus delbrueckii* subsp. *bulgaricus*) and *Streptococcus thermophilus,* optionally with additional microorganisms such as but not limited to probiotic species or other species that may provide desirable organoleptic or other qualities to the composition, e.g. *Lactococcus lactis.*

The present may also relate to the use of the inoculum as defined above for the preparation of a food product and/or to a method for the preparation of a fermented food product wherein this use and/or method comprises the step of adding the inoculum to milk and/or vegetal base.

### Methods for the preparation of fermented food products

The bacteria as provided herein are suitable for use in the preparation of fermented food products. Accordingly, in a third aspect the present invention also relates to the intended use of sucrose negative, stachyose and raffinose positive *Bifidobacteria* for the preparation of a food product.

In an embodiment, the use_of sucrose negative, stachyose and raffinose positive *Bifidobacteria* for the preparation of a food product comprises a step of adding the sucrose negative, stachyose and raffinose positive *Bifidobacteria* to milk and/or vegetal base.

In an embodiment, the use_of sucrose negative, stachyose and raffinose positive *Bifidobacteria* for the preparation of a food product comprises a step of adding the sucrose negative, stachyose and raffinose positive *Bifidobacteria* to milk and/or vegetal base.

In one embodiment the present invention provides a method for the preparation of a fermented food product comprising providing a mixture comprising one or more sucrose negative, stachyose and raffinose positive *Bifidobacteria* strains and fermenting. Preferably the sucrose negative, stachyose and raffinose positive *Bifidobacteria* are *B. breve* or *B. longum* , more preferably selected from the group consisting of CNCM 1-5542, CNCM 1-5543, CNCM I-5544 and/or combinations thereof.

Accordingly in one embodiment the present invention provides a method comprising the following steps:
i) providing a mixture comprising:
   a) milk and/or vegetal base
   b) one or more sucrose negative, stachyose and raffinose positive *Bifidobacteria* strains
ii) fermenting said mixture to provide a fermented food product.

Preferably the sucrose negative, stachyose and raffinose positive *Bifidobacteria* are *B. breve* or *B. longum,* more preferably selected from the group consisting of CNCM 1-5542, CNCM 1-5543, CNCM I-5544 and/or combinations thereof.

### i) Mixtures

Mixtures of the invention may prepared by mixing or otherwise combining a) milk and/or vegetal base with b) one or more sucrose negative, stachyose and raffinose positive *Bifidobacteria* strains.

Preferably, the mixture comprises at least about 30 % (w/w) milk and/or vegetal base, more preferably at least about 50% (w/w) milk and/or vegetal base and even more preferably at least about 70% (w/w) milk and/or vegetal base. In embodiments, the mixture comprises at 30 % to 99% (w/w) milk and/or vegetal base. In other embodiments, the mixture comprises 50% to 100% (w/w) milk and/or vegetal base. In other embodiments, the mixture comprises 70% to 100% (w/w) milk and/or vegetal base.

### ia) Base

In embodiments the milk and/or vegetal base disclosed herein comprise raffinose. In an embodiment raffinose is present in an amount between 50 and 200 mg/100g by weight of base, for example, or from 60 and 150 mg/100g, or from 70 and 100 mg/100g.

In embodiments the milk and/or vegetal base disclosed herein comprise stachyose. In an embodiment stachyose is present in an amount between 100 and 1000 mg/100g by weight of base, for example, or from 100 and 800 mg/100g, or from 150 and 700 mg/100g.

In embodiments the milk and/or vegetal base disclosed herein comprise sucrose. In an embodiment sucrose is present in an amount between 1% and 15% by weight, for example, or from 1% to 10%, or from 2% to 10% or from 3% to 10% or from 5% to 10% or from 5% to 9% or from 5% to 8% or from 5% to 7%. In embodiments the sucrose is provided to the base in the form of added sugar. In embodiments the added sugar is selected from the group consisting of beet sugar, brown sugar, brown rice sugar, cane juice, cane syrup, cane sugar, caramel, coconut sugar, corn syrup solids, corn syrup, refined sugar, confectioner's sugar, date sugar, maple syrup, malt, molasses, raw sugar, sugar, honey, agave, and/or combinations thereof.

In embodiments the milk and/or vegetal base disclosed herein comprise raffinose and sucrose as described herein.

In embodiments the milk and/or vegetal base disclosed herein comprise stachyose, raffinose and sucrose as described herein.

### ia) Vegetal base

In one embodiment, the vegetal base is an aqueous suspension comprising water and plant-matter selected from the group consisting of legumes, nuts, seeds, cereals and/or combination thereof.

In embodiments the vegetal base comprises water. Water is typically present in an amount balancing the amounts of other ingredients to 100% by weight. In an embodiment water is present in an amount between 40% and 99% by weight, for example, or from 60% to 90%, or from 40% to 45% or from 45% to 50% or from 50% to 55% or from 55% to 60% or from 60% to 65% or from 65% to 70% or from 70% to 75%, or from 75% to 80% or from 80% to 85% or from 80% to 85%. In one embodiment the water quality is monitored to ensure sufficiently low level of cations to ensure emulsification stability is not impacted. The total cation content can be from about 60 ppm (40 ppm for divalent ions and 20 ppm for monovalent ions) and the hardness of water can be 6 gram/gallon or less.

In other embodiments, the plant-matter comprises legumes, and most preferably, pulse or pulses. In other embodiments, the pulses are selected from the group consisting of split peas, field peas, dry peas, lentil, chickpeas, garbanzo bean, faba, konda, navy bean, white navy bean, white pea bean, pea bean, cow pea, horse bean, haricot, pinot bean, mottled bean, small red bean, red Mexican bean, kidney bean, black bean, black turtle bean, cranberry bean, roman bean, speckled sugar bean, lima bean, haba bean, Madagascar bean, green gram, mung bean, green bean, black gram, urad dal, soy and/or lupin. In preferred embodiments, the pulses are pea and/or chickpea.

In other embodiments, the nuts are selected from the group consisting of almonds, cashews, pecans, macadamias, hazelnuts, pistachio, walnuts or combinations thereof.

In other embodiments, the seeds are selected from the group consisting of hemp, pumpkin, quinoa, sesame, tiger nut, flax, chia, sunflower, coconut or combinations thereof.

In other embodiments, said cereals are selected from the group consisting of wheat, rye, spelt, barley, oat, millet, sorghum, rice, teff and combinations thereof.

Processes for the preparation of such suspensions are known in the art and typically comprise mechanical and/or enzymatic disruption of the plant-matter and hydration and/or combination with a solution, followed by mechanical separation of an aqueous fraction from starchy and/or fibrous matter, e.g., by decantering, centrifugation or filtration.

For example, the plant-matter may be milled, ground, soaked, dehulled, mixed with water, optionally enzymatic hydrolysed and/or homogenized etc. in order to produce a suitable aqueous composition.

In other embodiments, the plant matter may be a seed or nut butter such as sunflower, sesame, soy, almond, cashew, hazelnut or peanut butter. Processes for the preparation of nut butters typically comprise wet or dry grinding roasted or unroasted nuts to a paste having a particle size suitable for the preparation of nut beverages.

In other embodiments, the plant matter may be a hydrolyzed cereal suspension such as an oat milk or syrup. Processes for the preparation of such cereal suspensions typically comprise mixing an oat material (such as rolled oats, milled oats, oat flour or oatmeal) with water and treated enzymatically by amylases to hydrolyze starch followed by removal of suspended matter.

### ia) Milk base

Preferably, the milk base is animal milk, more preferably goat, ewe, camel, mare or cow milk, and most preferably to cow milk.

Preferably, the milk comprises one or more of skimmed, partially-skimmed or non-skimmed milk. Preferably, the milk or milks may be in liquid, powdered and/or concentrated form. In one embodiment, the milk further comprises milk components preferably selected from the group consisting of cream, casein, caseinate (for example calcium or sodium caseinate), whey proteins notably in the form of a concentrate (WPC), milk proteins in the form of a concentrate (MPC), milk protein hydrolysates, and mixtures thereof. In one embodiment, the milk or milks may be enriched or fortified with further milk components or other nutrients such as but not limited to vitamins, minerals, trace elements or other micronutrients.

### ia) Milk and/or vegetal base preparation

In embodiments the milk and/or vegetal base may be adjusted to a pre-defined standard prior to the preparation of the composition. Preferably said standard is a defined nutritional standard of proteins, carbohydrates, fats and/or micronutrients. Standardization of substances may be carried out by the addition of milk and/or vegetal base components to reach said predefined standard.

Preferably fermented products are prepared using milk and/or vegetal base that has been subjected to heat treatment at least equivalent to pasteurization to ensure safety of consumption of the final product in regard to pathogens. Preferably said heat treatment is carried out prior to the preparation of the composition.

Methods for carrying out such heat treatments are known to the one skilled in the art and include, pasteurization, ultra-heat treatment and sterilization. The selection of appropriate time and temperature combinations is within the scope of the skilled person and is selected according to the final product properties and shelf-life requirements.

In one embodiment the base preparation is carried out by means of the following successive steps:
1) standardization of nutritional levels of the raw material so as to obtain a standardized substance,
2) an optional stage of enrichment with dried matter of the standardized substance obtained in the preceding stage, so as to obtain an enriched substance,
3) preheating of the substance, so as to obtain a starting substance,
4) pasteurization and holding of the starting substance obtained in the preceding stage, so as to obtain a pasteurized and held substance,
5) an optional stage of homogenization of the pasteurized and held substance obtained in the preceding stage, so as to obtain a pasteurized, held and optionally homogenized substance,
6) initial cooling of the pasteurized, held and optionally homogenized substance obtained in the preceding stage, so as to obtain a pasteurized starting substance that has been held, optionally homogenized, and cooled down.

As used herein "standardization of substances" is taken to mean a stage of bringing the quantity of a given substance present in the starting substance to a pre-determined level.

As used herein "holding" is taken to mean a rapid heating and maintenance of temperature of the base and makes it possible to destroy the vegetative microbial flora, including pathogenic forms. Its typical duration is from 4 to 10 minutes, in particular from 5 to 8 minutes, and in particular approximately 6 minutes.

As used herein "homogenization" is taken to mean the dispersion of the fatty substances in the milk-type substance into small fat globules. The homogenization is carried out for example at a pressure of 100 to 280 bars, in particular 100 to 250 bars, in particular 100 to 200 bars, in particular approximately 200 bars. This homogenization stage is purely optional. It is in particular absent from the production method of products with 0% fatty substances.

### ib) sucrose negative, stachyose and raffinose positive Bifidobacteria

The mixture further comprises one or more sucrose negative, stachyose and raffinose positive *Bifidobacteria* strains. In embodiments the sucrose negative, stachyose and raffinose positive *Bifidobacteria* are selected from the group consisting of *Bifidobacterium animalis* (for example *Bifidobacterium animalis* subsp. *animalis* or *Bifidobacterium animalis subsp. lactis*), *Bifidobacterium longum, Bifidobacterium breve* and *Bifidobacterium bifidum.* Preferably the sucrose negative, stachyose and raffinose positive *Bifidobacteria* are *B. breve* or *B. longum* , more preferably selected from the group consisting of CNCM 1-5542, CNCM 1-5543, CNCM I-5544 and/or combinations thereof.

According to a further embodiment of the method for the preparation of a fermented food product as defined above, the mixture further comprises c) at least one, two, three or more strains of lactic acid bacteria.

The selection of suitable lactic acid bacteria strains is within the scope of the skilled person and is typically a thermophillic lactic acid bacteria. Examples of lactic acid bacteria that can be used include but are not limited to *Lactobacilli* (for example *Lactobacillus acidophilus, Lactobacillus buchneri, Lactobacillus delbruckeii,* in particular *L. delbrueckii subsp. bulgaricus* or *lactis, Lactobacillus casei, Lactobacillus plantarum, Lactobacillus reuteri, Lactobacillus johnsonii, Lactobacillus helveticus, Lactobacillus brevis, Lactobacillus rhamnosus*); *Lactococci* (for example *Lactococcus lactis,* typically *Lactococcus lactis subsp. lactis* or *Lactococcus lactis* subsp. *cremoris*)*.* Typically a mixture or association of a plurality of species of lactic acid bacteria may be used, typically a mixture or association of *Lactobacillus* and *Streptococcus.* For the preparation of yogurt or plant-based alternatives thereto this typically includes *Lactobacillus bulgaricus* (also referred to as *Lactobacillus delbrueckii* subsp. *bulgaricus*), optionally with additional microorganisms such as but not limited to probiotic species or other species that may provide desirable organoleptic or other qualities to the composition, e.g. *Lactococcus lactis.*

Accordingly in one embodiment the mixture further comprises at least one strain of *Lactobacillus bulgaricus* and optionally one or more strains of *Lactococcus lactis* and/or *Bifidobacterium.*

### ii) Fermentation

Typically a fermented product is prepared by culture of a substrate at a suitable temperature with suitable microorganisms to provide a reduction in pH, preferably to a pH equal to or lower than 5, preferably between about 3 and 4.7; more preferably between about 3.5 and about 4.7. The pH can be adjusted by controlling the fermentation by the microorganism and stopping it when appropriate, for example by cooling.

Suitable temperatures for such fermentation are typically about 36°C to about 44°C and the temperature is maintained for an incubation time sufficient to provide the desired reduction in pH. For the preparation of a fermented food product the temperature at the start of fermentation is typically about 36°C to about 43°C, in particular about 37°C to about 40°C, the temperature at the end of fermentation is typically about 37°C to about 44°C, in particular about 38°C to about 41°C. The fermentation time is typically about 6 to about 11 hours.

Subsequent to the fermentation the fermented product is cooled. Optionally a stage of intermediate cooling of the fermented food product may be performed to provide a pre-cooled fermented food product having a temperature of between about 22°C and about 4°C. Typically the intermediate cooling time is about 1 hour to about 4 hours, in particular about 1 hour 30 minutes to about 2 hours. The pre-cooled fermented food product is typically stored for up to 40 hours or less.

Preferably a stage of final cooling of the fermented product is performed such that the temperature at the start of the final cooling is less than about 22°C and the temperature at the end of the final cooling is about 4°C to about 10°C. The cooled product may then be stored, transported and/or distributed at a temperature from about 1°C to about 10°C for at least about 30 days, at least about 60 days or at least about 90 days.

According to a further embodiment, the method for the preparation of a fermented food product as defined above optionally comprises a stage of stirring at a pressure of at least 20 bars, or performing a dynamic smoothing, to obtain a composition having the desired viscosity, typically a viscosity of up to 20 mPa.s. Stirring or dynamic smoothing operations provide some shear to composition that typically allow a viscosity drop. Such operations are known by the one skilled in the art, and can be operated with conventional appropriate equipment. This stage is typically performed at cold temperature, for example at a temperature of from 1 °C to 20°C. Without intending to be bound to any theory, it is believed that applying some shear at cold temperature, typically by stirring at high pressure or by performing a dynamic smoothing, can lead to a fluid gel formation within the composition, that provides improved stability even at a low viscosity of up to 20 mPa.s.

Alternatively, according to a further embodiment, the method for the preparation of a fermented food product as defined above optionally comprises a stage of acid whey or plant-based whey alternative removal to provide a "strained fermented food composition". In this step an acid whey composition is separated from the curd resulting from the protein coagulation due to acidification during fermentation. Thus, one obtains:
- a fermented food product, typically comprising the proteins coagulum, referred to as a strained fermented food product, and
- an acid whey or plant-based whey alternative by-product

Such separation steps are known by the one skilled in art, for example in processes of making "greek yogurts". The separation can for example be carried out by reverse osmosis, ultrafiltration, or centrifugal separation. The separation step can be performed for example at a temperature of from 30°C to 45°C.

According to a further embodiment, the method for the preparation of a fermented food product as defined above optionally comprises a stage of addition of an intermediate preparation as described above prior or subsequent to fermentation, said intermediate preparation typically comprising a preparation of fruits and/or cereals and/or additives such as flavorings and/or colourings.

In embodiments the fermented food product of the invention and fermented food products prepared according to the method of the invention comprise raffinose. In an embodiment raffinose is present in an amount between 0.1 and 50 mg/100g by weight of fermented composition, for example, or from 2 and 40 mg/100g, or from 3 and 30 mg/100g.

In embodiments the fermented food product of the invention and fermented food products prepared according to the method of the invention comprise stachyose. In an embodiment stachyose is present in an amount between 100 and 1000 mg/100g by weight of base, for example, or from 100 and 800 mg/100g, or from 150 and 700 mg/100g.

In embodiments the fermented food product of the invention and fermented food products prepared according to the method of the invention comprise sucrose. In an embodiment sucrose is present in an amount between 1% and 15% by weight, for example, or from 1% to 10%, or from 2% to 10% or from 3% to 10% or from 5% to 10% or from 5% to 9% or from 5% to 8% or from 5% to 7%. In embodiments the sucrose is present fully or partially in the form of added sugar. In embodiments the added sugar is selected from the group consisting of beet sugar, brown sugar, brown rice sugar, cane juice, cane syrup, cane sugar, caramel, coconut sugar, corn syrup solids, corn syrup, refined sugar, confectioner's sugar, date sugar, maple syrup, malt, molasses, raw sugar, sugar, honey, agave, and/or combinations thereof.

In embodiments the fermented food product of the invention and fermented food products prepared according to the method of the invention comprise stachyose, raffinose and sucrose as described herein.

The invention will be further illustrated by the following non-limiting Figures and Example.

### Description of the figures

Figure 1 provides the raffinose and stachyose ("alpha-galactosides") degradation of the strains CNCM I- 5542, CNCM I- 5543 & CNCM I- 5544 according to Example 2.
Figure 2 provides the soy acidification curves of the strains CNCM I- 5542, CNCM I- 5543 & CNCM I-5544 according to Example 3.
Figure 3 provides the combined raffinose and stachyose ("alpha-galactosides") degradation of the strains CNCM I- 5542, CNCM I- 5543 & CNCM I- 5544 according to Example 3.
Figure 4 provides the soy acidification curves according to Example 4.

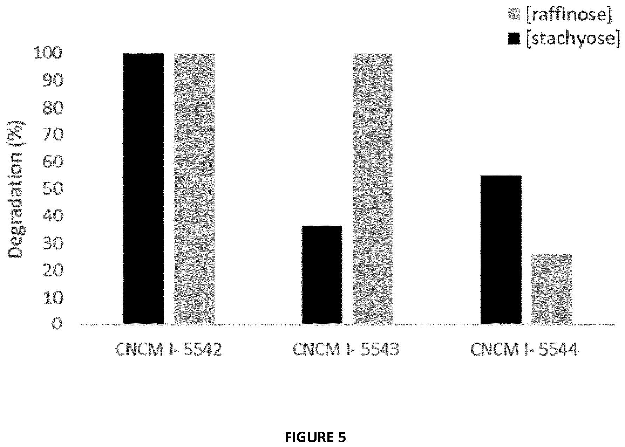

Figure 5 provides the degradation of alpha-galactosides in soy fermentation (HPLC quantification) according to Example 4.

### Examples

### Example 1: Screening of metabolic properties of Bifidobacteria strains

222 *Bifidobacteria* strains from the Applicant's Danone Culture Collection were screened to identify strains having a rare combination of two metabolic properties: the ability to metabolize raffinose and an absence of sucrose metabolization properties.

Raffinose metabolization screening: Each individual *Bifidobacteria* strain was tested for raffinose metabolization by screening for growth in a MRS no-sugar media supplemented with raffinose and pH indicator bromocresol purple, each strain was cultured at 37°C for 24 hours. Growth was detected by an observable colour change indicating acidification (i.e growth). Strains capable of growth in the raffinose media were then tested for growth in a media where the sole source of sugars was sucrose.

Sucrose metabolization screening: Each individual *Bifidobacteria* strain was tested for sucrose metabolization by screening for growth in a MRS no-sugar media supplemented with sucrose and pH indicator bromocresol purple, each strain was cultured at 37°C for 24 hours. Growth was detected by an observable colour change indicating acidification (i.e growth).

Only 13 strains of the tested 222 strains had the target metabolic phenotype ("RAF+SUC-"):

**Table 1**

| Species | No. of RAF+SUC- strains |
|---|---|
| *B. breve* | 5 |
| *B. longum* | 2 |
| *B. animalis* | 1 |
| *B. adolescentis* | 1 |
| *B. pseudocatenulatum* | 1 |

### Example 2: Rate of degradation of raffinose & stachyose

The 13 candidate strains identified in Example 1 were tested to identify strains having good rates of raffinose & stachyose degradation in MRS media.

### Method

Enzymatic tests were done on each strain's growth in 1) MRS no-sugar medium supplemented with raffinose 2) MRS no-sugar medium supplemented with stachyose. Raffinose & stachyose degradation in the tested growth media by the tested strains was measured using an enzymatic kit (K-RAFGA Megazyme).

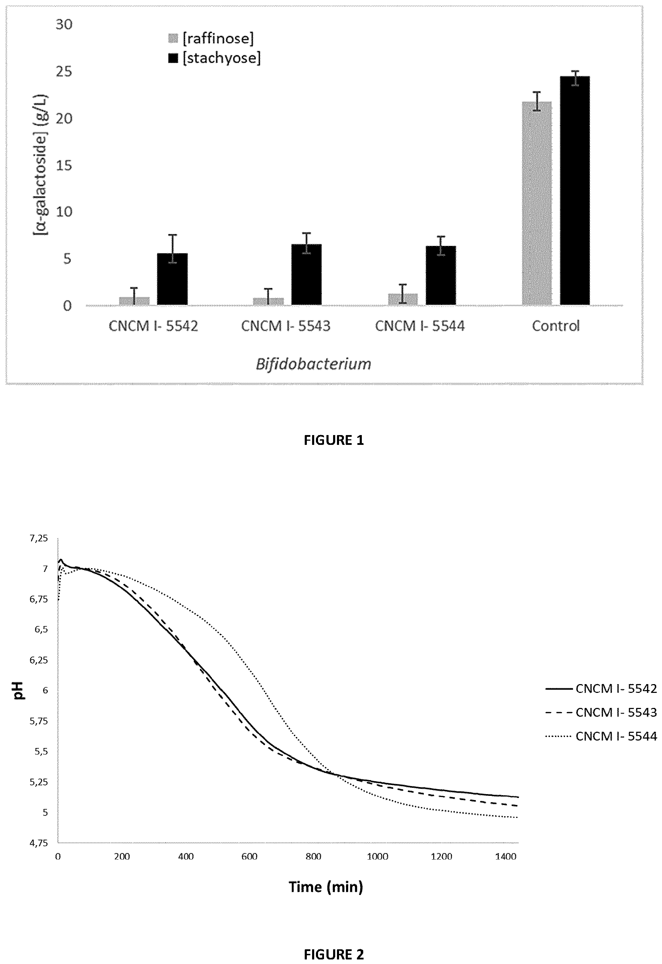

Figure 1 provides the raffinose and stachyose degradation of the strains CNCM I- 5542, CNCM I- 5543 & CNCM I- 5544 (control is the level of raffinose and stachyose in the media without the addition of any strains). The strains of the invention are efficient at degrading raffinose & stachyose and were accordingly considered good candidates for preparation of dairy-analogue plant-based products in products containing these. This was confirmed in Example 3.

### Example 3: Soy milk fermentation using CNCM I- 5542, CNCM I- 5543 & CNCM I- 5544

In order to determine the suitability of the strains for preparing fermented plant-based products the strains were tested for soy milk fermentation (which contains significant amounts of alpha-galactosides).

Commercially available soy milk (organic, no added sugar) was inoculated with 1% volume of the test strains. The mixtures were fermented at 37°C and monitored using a CINAC pH probe for 24 hours. Fermentation was then stopped by cooling and products stored at 10°C.

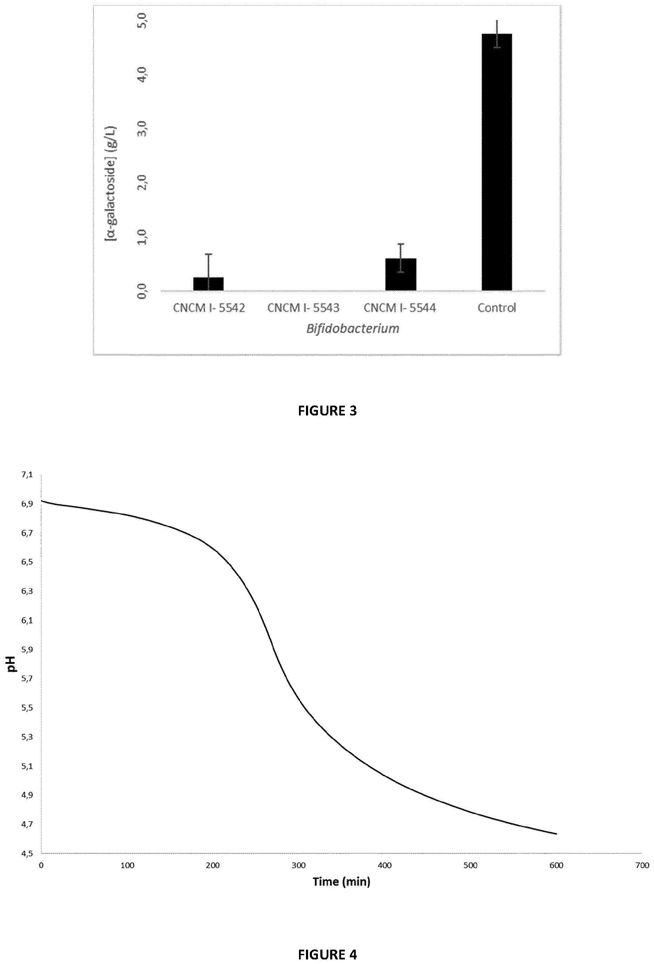

Figure 3 provides the raffinose and stachyose degradation of the strains CNCM I- 5542, CNCM I- 5543 & CNCM I- 5544 (control is the level of raffinose and stachyose in the soy milk without the addition of any strains).

Strains CNCM I- 5542, CNCM I- 5543 & CNCM I- 5544 were considered good candidates for preparation of dairy-analogue plant-based products, due to the excellent soy fermentation properties as determined by the acidification curves of Figure 2. It was observed that some strains of Table 1, while efficient at degrading raffinose and stachyose in the MRS media were not efficient at degrading these alpha-galactosides in the soy milk. This indicates that although 13 strains had the relevant metabolic phenotype, that the strains of the invention are surprisingly advantageous for the preparation of food products. Strain CNCM I- 5542 was selected as a prototype for the preparation of a plant-based probiotic yogurt-type dairy-analogue, using yogurt cultures (*L. bulgaricus* + *S. thermophilus*)*.*

### Example 4: Preparation of a plant-based probiotic yogurt-type dairy-analogue using CNCM 1-5542

A plant-based probiotic yogurt-type dairy-analogue was prepared by inoculating commercially available soy milk with 0.6% volume *L. bulgaricus* CNCM 1-5288, with 0.04% volume *S*. *thermophilus* CNCM I-1520 and with 0.06% volume *B. breve* CNCM I-5542 respectively precultured in MRS, Elliker and MRS containing 0.3g/L cysteine medium. The mixtures were fermented at 37°C and monitored using a CINAC pH probe until a pH of 4.7 was reached. Fermentation was then stopped by cooling and products stored at 10°C.

The combination of CNCM I-5542 together with yogurt cultures, was confirmed as a good candidate for preparation of dairy-analogue plant-based products, from the acidification curves of Figure 4.

Degradation of raffinose and stachyose by the strains was further confirmed using HPLC analysis. A Waters Ultrahydrogel^{®} DP120 Column was used at 0,5 ml/min with water as eluant. Refractive Index was used for the detection.

Sensory evaluation of the product was carried out by a trained tasting panel who evaluated the dairy notes of the flavour profile and a global sensory profile of the product. The addition of the strain CNCM I-5542 of the invention changed the flavour profile of the fermented product. When the *B. breve* was omitted the flavor profile was a fresh smell and pleasantly taste, with a note of cardboard. The addition of the *B. breve* resulted in a product that had a neutral smell and more dairy notes in the mouth. Surprisingly thus strain CNCM I-5542 contributed to a more dairy-like organoleptic experience in the yogurt alternative product.

## Claims

1. A *Bifidobacteria* strain **characterized in that** it is sucrose negative, stachyose and raffinose positive.

2. The strain of claim 1 further **characterized in that** it is a *Bifidobacteria* species, selected from the group consisting *of Bifidobacterium animalis, Bifidobacterium longum, Bifidobacterium breve* and *Bifidobacterium bifidum.*

3. A *Bifidobacteria* strain deposited at the CNCM under reference number CNCM 1-5542.

4. A *Bifidobacteria* strain deposited at the CNCM under reference number CNCM 1-5543.

5. A *Bifidobacteria* strain deposited at the CNCM under reference number CNCM 1-5544.

6. A composition comprising one or more strains according to any one of claims 1-5 and/or combinations thereof.

7. The composition according to claim 6 comprising at least 10⁵ CFU/g of said strain.

8. The composition of claim 6 or 7 wherein said composition is a food product, optionally a fermented food product.

9. The composition of claim 8 wherein said food product is a dairy product or a plant-based product.

10. A method for the preparation of a fermented food product comprising:
i) providing a mixture comprising a) milk and/or vegetal base, and b) at least one strain as defined in any one of claims 1-5 and/or combinations thereof,
ii) fermenting said mixture to provide a fermented food product.

11. The method of claim 10 wherein the base a) comprises raffinose and sucrose.

12. The method of claim 10 or 11 wherein the base a) comprises iii) at least one strain of lactic acid bacteria.

13. A fermented food product obtainable according to the method of claims 10-12.

14. The composition of any one of claims 6-9 or the product obtainable according to the method of claims 10-12 comprising at least 1-10% w/w sucrose.

15. The composition of any one of claims 6-9 or the product obtainable according to the method of claims 10-12 having a pH of 4.7 or lower.

16. The composition of any one of claims 6-9 or the product obtainable according to the method of claims 10-12 further comprising at least one strain of lactic acid bacteria.
